(19) Europäisches Patentamt
European Patent Office
Office européen des brevets

(11) Veröffentlichungsnummer: **0 302 375 B1**

(12) **EUROPÄISCHE PATENTSCHRIFT**

(45) Veröffentlichungstag der Patentschrift: **15.04.92**

(51) Int. Cl.5: **C07C 45/50**

(21) Anmeldenummer: **88112112.3**

(22) Anmeldetag: **27.07.88**

# (54) Verfahren zur Herstellung von Aldehyden.

(30) Priorität: **06.08.87 DE 3726128**

(43) Veröffentlichungstag der Anmeldung:
**08.02.89 Patentblatt 89/06**

(45) Bekanntmachung des Hinweises auf die Patenterteilung:
**15.04.92 Patentblatt 92/16**

(84) Benannte Vertragsstaaten:
**AT BE DE FR GB IT NL SE**

(56) Entgegenhaltungen:
**FR-A- 2 314 910**
**US-A- 3 928 232**
**US-A- 4 578 523**

(73) Patentinhaber: **HOECHST AKTIENGESELLSCHAFT**
**Postfach 80 03 20**
**W-6230 Frankfurt am Main 80(DE)**

(72) Erfinder: **Bach, Hanswilhelm, Dr. Dipl.-Chem.**
**Alleestrasse 56**
**W-4100 Duisburg 11(DE)**
Erfinder: **Bahrmann, Helmut, Dr. Dipl.-Chem.**
**Rohstrasse 48**
**W-4236 Hamminkeln Brünen(DE)**
Erfinder: **Cornils, Boy, Dr. Dipl.-Chem.**
**Kirschgartenstrasse 6**
**W-6238 Hofheim(DE)**
Erfinder: **Gick, Wilhelm, Dr. Dipl.-Chem.**
**Im Buschhuck 8**
**W-4100 Duisburg 74(DE)**
Erfinder: **Heim, Volker**
**Schützenstrasse 6**
**W-4200 Oberhausen(DE)**
Erfinder: **Konkol, Werner, Dr. Dipl.-Chem.**
**Lützowstrasse 40a**
**W-4200 Oberhausen 11(DE)**
Erfinder: **Wiebus, Ernst**
**Ferdinandstrasse 77**
**W-4200 Oberhausen 11(DE)**

EP 0 302 375 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von Aldehyden durch Hydroformylierung von Olefinen in Gegenwart wasserlöslicher Rhodium-Komplexkatalysatoren.

Es ist bekannt, durch Umsetzung von Olefinen mit Kohlenmonoxid und Wasserstoff Aldehyde und Alkohole herzustellen. Die Reaktion wird durch Hydridometallcarbonyle, vorzugsweise solcher der Metalle der 8. Gruppe des Periodensystems, katalysiert. Neben Kobalt, das als Katalysatormetall in großem Umfang technische Anwendung findet, gewinnt in letzter Zeit Rhodium zunehmend Bedeutung. Im Gegensatz zu Kobalt gestattet Rhodium, die Reaktion bei niedrigem Druck durchzuführen; darüber hinaus werden vorzugsweise geradkettige n-Aldehyde und nur in untergeordnetem Maße iso-Aldehyde gebildet. Schließlich ist auch die Hydrierung der Olefine zu gesättigten Kohlenwasserstoffen bei Anwendung von Rhodium-Katalysatoren deutlich niedriger als bei Anwendung von Kobalt-Katalysatoren.

Bei den in der Technik eingeführten Verfahren wird der Rhodium-Katalysator in Form von modifizierten Hydridorhodiumcarbonylen eingesetzt, die zusätzliche und gegebenenfalls überschüssige Liganden enthalten. Besonders bewährt haben sich als Liganden tertiäre Phosphine oder Phosphite. Ihre Anwendung ermöglicht, den Reaktionsdruck auf Werte unter 30 MPa zu senken.

Probleme wirft bei diesem Verfahren jedoch die Abtrennung der Reaktionsprodukte und die Wiedergewinnung der im Reaktionsprodukt homogen gelösten Katalysatoren auf. Im allgemeinen destilliert man hierzu das Umsetzungsprodukt aus dem Reaktionsgemisch ab. In der Praxis kann dieser Weg wegen der thermischen Empfindlichkeit der gebildeten Aldehyde und Alkohole aber nur bei der Hydroformylierung niedriger Olefine, d.h. Olefine mit bis zu etwa 8 Kohlenstoffatomen im Molekül, beschritten werden. Außerdem hat sich gezeigt, daß die thermische Belastung des Destillationsgutes auch zu erheblichen Katalysatorverlusten durch Zersetzung der Rhodium-Komplexverbindungen führt.

Die geschilderten Mängel werden durch Anwendung von Katalysatorsystemen vermieden, die in Wasser löslich sind. Derartige Katalysatoren sind z.B. in der DE-PS 26 27 354 beschrieben. Die Löslichkeit der Rhodium-Komplexverbindungen wird hierbei durch Verwendung von Sulfonierten Triarylphosphinen als Komplexbestandteil erreicht. Die Abtrennung des Katalyators vom Reaktionsprodukt nach Beendigung der Hydroformylierungsreaktion erfolgt bei dieser Verfahrensvariante einfach durch Trennung von wäßriger und organischer Phase, d.h. ohne Destillation und damit ohne zusätzliche thermische Verfahrensschritte. Ein weiteres Merkmal dieser Arbeitsweise ist, daß mit hoher Selektivität aus endständigen Olefinen n-Aldehyde und nur in ganz untergeordnetem Maße iso-Aldehyde gebildet werden. Als Komplexbestandteile wasserlöslicher Rhodium-Komplexverbindungen werden neben sulfonierten Triarylphosphinen auch carboxylierte Triarylphosphine eingesetzt.

Die bekannten Zweiphasen-Verfahren haben sich ausgezeichnet bei der Hydroformylierung niederer Olefine, insbesondere Ethylen und Propylen, bewährt. Setzt man höhere Olefine wie Octen oder Decen ein, gehen der Umsetz und/oder die Selektivität zu n-Verbindungen merklich zurück. Die Wirtschaftlichkeit der Umsetzung in technischem Maßstab ist dann häufig nicht mehr gegeben.

Zur Behebung dieser Schwierigkeiten hat man verschiedene Wege beschritten. Nach der DE 34 12 335 Al setzt man bei einem Verfahren zur Herstellung von Aldehyden durch Umsetzung von Olefinen mit Kohlenmonoxid und Wasserstoff in flüssiger Phase und in Gegenwart von Wasser und wasserlöslichen Rhodium-Phosphin-Komplexverbindungen dem Reaktionsmedium einen Lösungsvermittler zu. Ein Nachteil dieser Arbeitsweise ist die Verwendung von Reagenzien, die nicht reaktionseigen sind, d.h. nicht zu den Ausgangsstoffen, den Reaktionsprodukten oder den katalytisch wirksamen Substanzengehören, so daß ein negativer Einfluß auf das Reaktionsgeschehen und insbesondere auf die Lebensdauer der Katalysatoren nicht ausgeschlossen werden kann.

Der in der DE 34 20 491 Al beschriebene Prozeß zur Hydroformylierung von Olefinen verwendet katalytisch aktive Rhodium-Komplexverbindungen, in denen die Komplexliganden quartäre Ammoniumsalze sulfonierter Triarylphosphine sind. Die quartären Ammoniumionen enthalten einen Alkyl- oder Aralkylrest mit 7 bis 18 Kohlenstoffatomen und 3 geradkettige oder verzweigte Alkylreste mit 1 bis 4 Kohlenstoffatomen. Dieses Verfahren hat sich auch bei der Umsetzung höherer Olefine sehr bewährt. Seiner allgemeinen Anwendung steht jedoch der hohe Preis der quartären Ammoniumhydroxide entgegen, die zur Gewinnung der quartären Ammoniumsalze sulfonierter Triarylphosphine erforderlich sind.

Es bestand daher die Aufgabe, ein Verfahren zu entwickeln, das die aufgezeigten Mängel vermeidet und sicherstellt, daß auch höhere Olefine in guter Ausbeute zu n-Aldehyden umgesetzt werden.

Die Erfindung besteht in einem Verfahren zur Herstellung von Aldehyden durch Umsetzung von Olefinen mit sechs und mehr Kohlenstoffatomen mit Kohlenmonoxid und Wasserstoff in flüssiger Phase in Gegenwart von Wasser sowie Rhodium in metallischer Form oder als Verbindung und von wasserlöslichen Arylphosphinen bei Temperaturen von 20 bis 150 °C und Drücken von 0,1 bis 20 MPa. Es ist dadurch

gekennzeichnet, daß als wasserlösliche Phosphine ein Gemisch aus Alkalisalzen und 1 bis 30 Mol-% (bezogen auf das Phosphingemisch) quartären Ammoniumsalzen der allgemeinen Formel

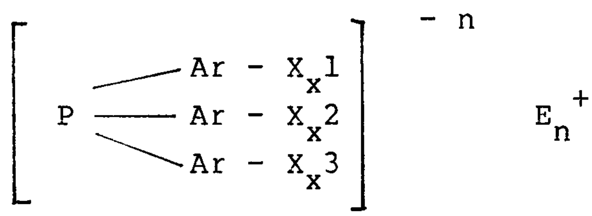

eingesetzt wird, wobei Ar für einen Arylrest und X für eine Sulfonsäuregruppe steht, $x^1$, $x^2$, $x^3$ 0 oder 1 bedeuten mit der Maßgabe, daß mindestens eine Zahl $x^1$, $x^2$ oder $x^3$ 1 ist, E ein Alkalimetallion oder ein quartäres Ammoniumion der allgemeinen Formel

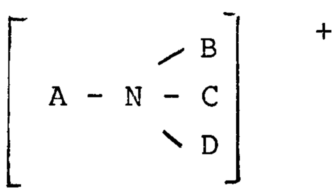

ist, in der A für einen Alkylrest mit 6 bis 20 Kohlenstoffatomen steht und B, C, D geradkettige oder verzweigte Alkylreste mit 1 bis 4 Kohlenstoffatomen sind und n eine ganze Zahl zwischen 1 und 3 ist.

Überraschenderweise hat sich gezeigt, daß es nicht erforderlich ist, ausschließlich mit quartären Ammoniumsalzen sulfonierter Triarylphosphine als Komplexliganden für das Rhodium zu arbeiten, um auch höhere Olefine mit sehr gutem Erfolg durch Hydroformylierung in Aldehyde zu überführen. Auch Gemische aus Alkalisalzen und geringen Mengen von quartären Ammoniumsalzen sind als Komplexliganden für das Rhodium geeignet.

Nach einer bevorzugten Ausführungsform des erfindungsgemäßen Verfahrens enthält das Gemisch der wasserlöslichen Phosphine 3 bis 15 und insbesondere 5 bis 10 Mol-% (bezogen auf das Phosphingemisch) quartäre Ammoniumsalze.

Zu den Alkalisalzen der wasserlöslichen, sulfonierten Triarylphosphine gehören auch die Ammoniumverbindungen, also Salze, die das Kation $NH_4^+$ enthalten. Besonders zweckmäßig ist es, mit Natrium- und/oder Kaliumsalzen zu arbeiten.

Die erfindungsgemäß eingesetzten quartären Ammoniumverbindungen zeichnen sich dadurch aus, daß sie einen kohlenstoffreichen Alkylrest und 3 kurzkettige Alkylreste enthalten. Sowohl der kohlenstoffreichere Rest als auch die kurzkettigen Alkylreste können unverzweigt, gegebenenfalls aber auch verzweigt sein. Bevorzugt werden quartäre Ammoniumionen, deren kohlenstoffreicher Rest, ein geradkettiger Alkylrest mit 10 bis 18 und insbesondere mit 12 bis 16 Kohlenstoffatomen ist. Kurzkettige Alkylreste sind vorzugsweise Methyl- und/oder Ethylreste.

Als wasserlösliche Phosphine der oben wiedergegebenen allgemeinen Formel werden gemäß der neuen Arbeitsweise insbesondere Verbindungen eingesetzt, in denen Ar ein Phenyl- oder Naphthylrest, insbesondere ein Phenylrest und die Summe von $x^1$, $x^2$ und $x^3$ 2 oder 3 ist.

Beispiele für wasserlösliche Phosphine, die sich zur Durchführung des neuen Verfahrens eignen, sind Natrium- und/oder Kalium-Triphenylphosphin-trisulfonate und Triphenylphosphin-disulfonate sowie Tetraalykammoniumsalze der genannten Triphenylphosphinsulfonate mit folgenden Kationen: Trimethylcetylammonium, Trimethyldodecylammonium, Trimethyltetradecylammonium, Trimethylhexadecylammonium, Dodecylethyldimethylammonium.

Zur Herstellung der in dem beanspruchten Verfahren verwendeten Phosphine geht man von sulfonierten Triarylphosphinen aus, die durch Behandlung von Triarylphosphinen mit Oleum erhalten werden. Durch Variation der Reaktionsbedingungen, insbesondere der Reaktionszeit, der Reaktionstemperatur und des Verhältnisses von Triarylphosphin zu Schwefeltrioxid lassen sich bevorzugt mono-, di- oder trisulfonierte Arylphosphine herstellen.

Zweckmäßig gewinnt man aus dem Sulfonierungsprodukt zunächst in Wasser unlösliche, in organischen Lösungsmitteln jedoch lösliche Aminsalze. Sie werden anschließend durch Behandlung mit einem quartären Ammoniumhydroxid in das gewünschte Onium-Salz des sulfonierten Triarylphosphins überführt.

Die Umsetzung der Olefine mit Wasserstoff und Kohlenmonoxid nach dem neuen Verfahren erfolgt bei Temperaturen von 20 bis 150 °C, insbesondere 50 bis 120 °C und Drucken von 0,01 bis 10 MPa, insbesondere 0,1 bis 5 MPa.

Der Katalysator kann dem Reaktionssystem präformiert zugesetzt werden. Mit gleich gutem Erfolg läßt er sich aber auch aus den Komponenten Rhodium oder Rhodiumverbindung und der wäßrigen Lösung des quartären Ammoniumsalzes des sulfonierten Triarylphosphins unter Reaktionsbedingungen im Reaktionsgemisch, also in Gegenwart des Olefins, herstellen. Außer metallischem Rhodium in feinverteilter Form können als Rhodiumquelle wasserlösliche Rhodiumsalze wie Rhodiumchlorid, Rhodiumsulfat, Rhodiumacetat oder in organischen Medien lösliche Verbindungen wie Rhodium-2-ethylhexanoat oder unlösliche Verbindungen wie Rhodiumoxide eingestzt werden.

Die Rhodiumkonzentration in der wäßrigen Katalysatorlösung beträgt 10 bis 2000 Gew.-ppm, vorzugsweise 50 bis 800 Gew.-ppm, bezogen auf die Lösung. Das quartäre Ammoniumsalz des sulfonierten Phosphins wird in einer solchen Menge eingesetzt, daß auf 1 Grammatom Rhodium 2 bis 300 Mol, vorzugsweise 1 bis 100 Mol, Phosphinverbindung kommen.

Der pH-Wert der wäßrigen Katalysatorlösung soll nicht unter 2 liegen. Allgemein stellt man einen pH-Wert von 2 bis 13, vorzugsweise 5 bis 7, ein.

Um den pH-Wert während der Hydroformylierung konstant zu halten, empfiehlt sich die Verwendung von aus Natriumacetat und Essigsäure bestehenden Pufferlösungen. Die Pufferlösung wird der wäßrigen Phase in einer Menge von 1 bis 20, vorzugsweise 3 bis 10 Gew.-%, bezogen auf die gesamte wäßrige Lösung, zugesetzt.

Die Zusammensetzung des Synthesegases, d.h. das Verhältnis von Kohlenmonoxid zu Wasserstoff, kann in weiten Grenzen variiert werden. Im allgemeinen setzt man ein Synthesegas ein, in dem das Volumverhältnis von Kohlenmonoxid zu Wasserstoff 1 : 1 beträgt oder von diesem Wert nur wenig abweicht.

Die Umsetzung kann sowohl absatzweise als auch kontinuierlich durchgeführt werden.

Das erfindungsgemäße Verfahren wird mit Erfolg bei der Hydroformylierung von geradkettigen oder verzweigten Olefinen mit acht und mehr, insbesondere mit zehn bis zwanzig Kohlenstoffatomen angewandt. Die Doppelbindung in diesen Olefinen kann end- oder innenständig sein.

Die folgenden Beispiele erläutern die Erfindung näher ohne sie auf die beschriebenen Ausführungsformen zu beschränken. Zur Charakterisierung der Leistungsfähigkeit der Katalysatorsysteme werden neben dem Verhältnis von n-Aldehyd zu i-Aldehyd die Begriffe "Aktivität", definiert als

$$A = \frac{\text{mole Aldehyde}}{\text{g-Atom Rh . min}}$$

und "Produktivität", definiert als

$$P = \frac{\text{g Aldehyd}}{\text{ml Katalysatorlösung x h}}$$

verwendet. Die Alkohol- und Kohlenwasserstoffbildung ist minimal.

Beispiele 1 bis 5

In einem mit Tauchstutzen versehenen 1 l Autoklaven werden die in Tabelle 1 angegebenen Mengen einer wäßrigen Lösung, die die Na-salze der Triphenylphosphin-m-disulfonsäure und der Triphenylphosphin-m-trisulfonsäure im Verhältnis 1 : 10 (in den Tabellen vereinfacht TPPTS-Na genannt) und, bis auf Beispiel 1, ein Oniumsalz enthält, sowie die jeweils angegebene Menge einer Pufferlösung (die z.B. 3 mol Natriumacetat und 0,3 mol Essigsäure enthält), sowie 400 ppm Rh als Rh-Acetat vorgelegt. Darauf wird Synthesegas ($CO/H_2$ = 1 : l) bis zu einem Druck von 2,5 MPa aufgepreßt. Die Reaktionslösung

wird bei 125 ° C 3 h unter Rühren mit dem Synthesegas behandelt. Man kühlt auf etwa 30 ° C, stellt die Rührung ab und drückt nach einer Absetzzeit von 15 Minuten die überschüssige Lösung über den Tauchstutzen heraus; sie wird analysiert. Die restliche Lösung verbleibt im Autoklaven. Zu ihr werden unter Rühren über eine Druckpumpe die in Tabelle 1 angegebenen Mengen n-Hexen-1 gepumpt. Unter Aufrechterhaltung eines Druckes von 2,5 MPa wird 3 Stunden auf 125 ° C erhitzt. Danach läßt man auf 30 ° C abkühlen und absitzen. Die überstehende organische Phase wird nach einer 15-minutigen Beruhigungsdauer über den Tauchstutzen herausgedrückt. Die organische Phase wird gewogen und gaschromatographisch untersucht. Das Ergebnis der Umsetzung ist als Durchschnitt von 5 Versuchen in der nachfolgenden Tabelle 2 aufgeführt.

**Tabelle 1**

| | Beispiel 1[a] | Beispiel 2[a] | Beispiel 3 | Beispiel 4 | Beispiel 5 |
|---|---|---|---|---|---|
| TPPTS-Na-Lsg. (g) | 413 | 431 | 438 | 440 | 560 |
| Puffer-Lsg. (g) | 23 | 25 | 31 | 25 | 30 |
| P(III)-Gehalt d.Katalysator-Lsg. (mol/kg) | 0,324 | 0,374 | 0,330 | 0,364 | 0,393 |
| Hexen-Menge (g) | 175 | 175 | 175 | 175 | 200 |

**Tabelle 2**

| Kennwerte der Reaktion | 100 mol-% TPPTS-Na | 92,5 mol-% TPPTS-Na + 7,5 mol-% TPPTS-NR4 | | | |
|---|---|---|---|---|---|
| | Beispiel 1[a] | Beispiel 2[a] | Beispiel 3 | Beispiel 4 | Beispiel 5 |
| | $Na^*$ | $Bu_4N^*$ | $C_{12}H_{25}Me_3N^*$ | $(C_{14}H_{29}Me_3N^*$ | $C_{16}H_{31}Me_3N^*$ |
| Umsatz (%) | 22 | 37 | 56 | 73 | 56 |
| Produktivität | 0,035 | 0,059 | 0,096 | 0,127 | 0,110 |
| Aktivität | 1,18 | 2,0 | 3,4 | 4,41 | 2,8 |
| n/i-Verhältnis | 98/2 | 98/2 | 92/8 | 93/7 | 91/9 |

Me = $-CH_3$
Bu = $-C_4H_9$

a) zum Vergleich.

## Patentansprüche

1. Verfahren zur Herstellung von Aldehyden durch Umsetzung von Olefinen mit sechs und mehr Kohlenstoffatomen mit Kohlenmonoxid und Wasserstoff in flüssiger Phase in Gegenwart von Wasser sowie

Rhodium in metallischer Form oder als Verbindung und von wasserlöslichen Arylphosphinen bei Temperaturen von 20 bis 150 °C und Drücken von 0,1 bis 20 MPa, dadurch gekennzeichnet, daß als wasserlösliche Phosphine ein Gemisch aus Alkalisalzen und 1 bis 30 Mol-% (bezogen auf das Phosphingemisch) quartären Ammoniumsalzen der allgemeinen Formel

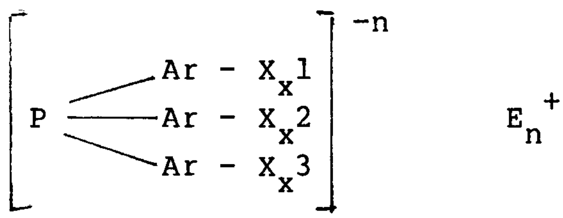

eingesetzt wird, wobei Ar für einen Arylrest und X für eine Sulfonsäuregruppe steht, $x^1$, $x^2$, $x^3$ 0 oder 1 bedeuten mit der Maßgabe, daß mindestens eine Zahl $x^1$, $x^2$ oder $x^3$ 1 ist, E ein Alkalimetallion oder ein quartäres Ammoniumion der allgemeinen Formel

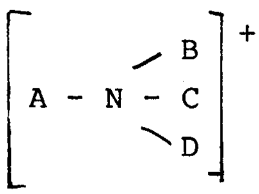

ist, in der A für einen Alkylrest mit 6 bis 20 Kohlenstoffatomen steht und B, C, D geradkettige oder verzweigte Alkylreste mit 1 bis 4 Kohlenstoffatomen sind und n eine ganze Zahl zwischen 1 und 3 ist.

2. Verfahren nach Anspruch 1, dadurch gekennzeichnet, daß das Gemisch der wasserlöslichen Phosphine 3 bis 15 und insbesondere 5 bis 10 Mol-% (bezogen auf das Phosphingemisch) quartäre Ammoniumsalze enthält.

3. Verfahren nach Anspruch 1 oder 2, dadurch gekennzeichnet, daß die Alkalisalze der wasserlöslichen Phosphine Natrium und/oder Kalium als Kation enthalten.

4. Verfahren nach einem oder mehreren der Ansprüche 1 bis 3, dadurch gekennzeichnet, daß in der allgemeinen Formel des quartären Ammoniumions A einen Alkylrest mit 10 bis 18 und insbesondere 12 bis 16 Kohlenstoffatomen bedeutet.

5. Verfahren nach einem oder mehreren der Ansprüche 1 bis 4, dadurch gekennzeichnet, daß in der allgemeinen Formel des quartären Ammoniumions B, C und D einen Methyl- und/oder Ethylrest bedeuten.

6. Verfahren nach Anspruch 4 und 5, dadurch gekennzeichnet, daß das quartäre Ammoniumion das Trimethyldodecylammoniumion, das Trimethyltetradecylammoniumion oder das Trimethylhexadecylammoniumion ist.

7. Verfahren nach einem oder mehreren der Ansprüche 1 bis 6, dadurch gekennzeichnet, daß die wäßrige, Rhodium und wasserlösliche Arylphosphine enthaltende Katalysatorlösung einen pH-Wert von 5 bis 7 aufweist.

8. Verfahren nach einem oder mehreren der Ansprüche 1 bis 7, dadurch gekennzeichnet, daß die Olefine acht und mehr, insbesondere zehn bis zwanzig Kohlenstoffatome enthalten.

**Claims**

**1.** Process for the preparation of aldehydes through reaction of olefins having six or more carbon atoms with carbon monoxide and hydrogen in the liquid phase in the presence of water and rhodium in metallic form or as a compound, and in the presence of water-soluble arylphosphines at temperatures from 20 to 150 °C and pressures from 0.1 to 20 MPa, characterized in that the water-soluble phosphines employed are a mixture of alkali metal salts and 1 to 30 mol-% of quaternary ammonium salts (relative to the phosphine mixture) of the general formula

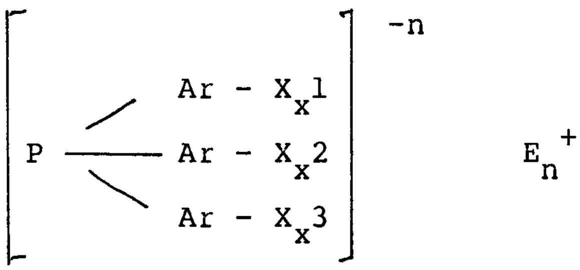

where Ar represents an aryl radical and X represents a sulphonic acid group, $x^1$, $x^2$ and $x^3$ denote 0 or 1, with the proviso that at least one number $x^1$, $x^2$ or $x^3$ is 1, E is an alkali metal ion or a quaternary ammonium ion of the general formula

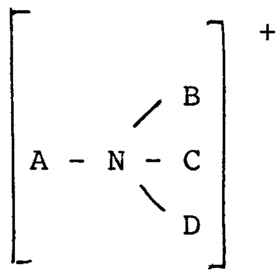

in which A represents an alkyl radical having 6 to 20 carbon atoms and B, C and D are straight-chain or branched alkyl radicals having 1 to 4 carbon atoms, and n is an integer between 1 and 3.

**2.** Process according to Claim 1, characterized in that the mixture of water-soluble phosphines contains 3 to 15 and in particular 5 to 10, mol-%, (relative to the phosphine mixture) of quaternary ammonium salts.

**3.** Process according to Claim 1 or 2, characterized in that the alkali metal salts of the water-soluble phosphines contain sodium and/or potassium as cation.

**4.** Process according to one or more of Claims 1 to 3, characterized in that, in the general formula of the quaternary ammonium ion, A denotes an alkyl radical having 10 to 18 and in particular 12 to 16 carbon atoms.

**5.** Process according to one or more of Claims 1 to 4, characterized in that, in the general formula of the quaternary ammonium ion, B, C and D denote methyl and/or ethyl radicals.

**6.** Process according to Claim 4 or 5, characterized in that the quaternary ammonium ion is the trimethyldodecylammonium ion, the trimethyltetradecylammonium ion or the trimethylhexadecylammonium ion.

**7.** Process according to one or more of Claims 1 to 6, characterized in that the aqueous catalyst solution containing rhodium and water-soluble arylphosphines has a pH from 5 to 7.

8. process according to one or more of Claims 1 to 7, characterized in that the olefins contain eight or more, in particular ten to twenty, carbon atoms.

**Revendications**

1. Procédé de préparation d'aldéhydes par réaction d'oléfines à 6 atomes de carbone et plus avec l'oxyde de carbone et l'hydrogène en phase liquide en présence d'eau et de rhodium à l'état métallique ou à l'état de composé et d'arylphosphines solubles dans l'eau à des températures de 20 à 150 °C et des pressions de 0,1 à 20 MPa, caractérisé en ce que l'on utilise en tant phosphines solubles dans l'eau un mélange de sels alcalins et de 1 à 30 mol% (par rapport au mélange des phosphines) de sels d'ammonium quaternaire de formule générale

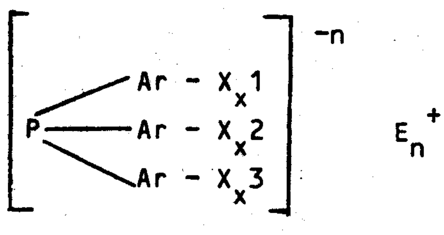

dans laquelle Ar représente un groupe aryle et X un groupe acide sulfonique, $x^1$, $x^2$, $x^3$ sont égaux à 0 ou 1 sous réserve que l'un au moins des indices $x^1$, $x^2$ ou $x^3$ est égal à 1, E représente un ion de métal alcalin ou un ion d'ammonium quaternaire de formule générale

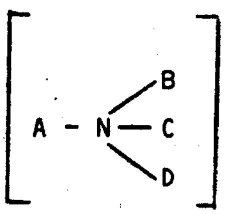

dans laquelle A représente un groupe alkyle en $C_6$-$C_{20}$ et B, C, D des groupes alkyle à chaîne droite ou ramifiée en $C_1$-$C_4$, et n est un nombre entier allant de 1 à 3.

2. Procédé selon la revendication 1, caractérisé en ce que le mélange des phosphines solubles dans l'eau contient de 3 à 15 et plus spécialement de 5 à 10 mol% (par rapport au mélange des phosphines) de sels d'ammonium quaternaire.

3. Procédé selon la revendication 1 ou 2, caractérisé en ce que les sels alcalins des phosphines solubles dans l'eau sont des sels de sodium et/ou de potassium.

4. Procédé selon une ou plusieurs des revendications 1 à 3, caractérisé en ce que, dans la formule générale de l'ion ammonium quaternaire, A représente un groupe alkyle en $C_{10}$-$C_{18}$ et plus spécialement en $C_{12}$-$C_{16}$.

5. Procédé selon une ou plusieurs des revendications 1 à 4, caractérisé en ce que, dans la formule générale de l'ion ammonium quaternaire, B, C et D représentent des groupes méthyle et/ou éthyle.

6. Procédé selon les revendications 4 et 5, caractérisé en ce que l'ion ammonium quaternaire est un ion triméthyldodécylammonium, triméthyltétradécylammonium ou triméthylhexadécylammonium.

7. Procédé selon une ou plusieurs des revendications 1 à 6, caractérisé en ce que la solution aqueuse de catalyseur contenant le rhodium et les arylphosphines solubles dans l'eau est à un pH de 5 à 7.

8. Procédé selon une ou plusieurs des revendications 1 à 7, caractérisé en ce que les oléfines

contiennent 8 atomes de carbone et plus, en particulier 10 à 20 atomes de carbone.